# EUROPEAN PATENT APPLICATION

(11) **EP 2 133 322 A1**
(43) Date of publication of application: **16.12.2009**
(21) Application number: 08158022.7
(22) Date of filing: 11.06.2008
(51) Int. Cl.: C07C 51/09, C07C 17/14, C07C 17/26, C07C 253/14, C07C 255/35, C07C 57/62, C07C 25/18

(54) **Process of preparing derivatives of 1-(2-halobiphenyl-4-yl)-cyclopropanecarboxylic acid**

(71) Applicant: CHIESI FARMACEUTICI S.p.A., 43100 Parma (IT)
(72) Inventor: Folleas, Benoit, 43100 Parma (IT); Botte, Hubert, 43100 Parma (IT); Delacroix, Thomas, 43100 Parma (IT); Pivetti, Fausto, 43100 Parma (IT)
(74) Representative: Minoja, Fabrizio

(57) **Abstract**

The invention relates to a process for preparing a compound according to formula (I)

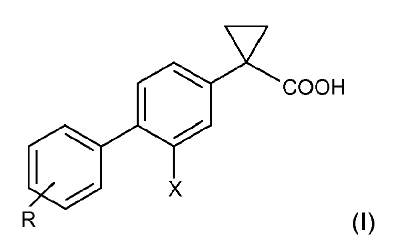

The invention also relates to intermediates useful in the process.

## Description

### TECHNICAL FIELD

The invention relates to a process for preparing a compound according to formula (I)

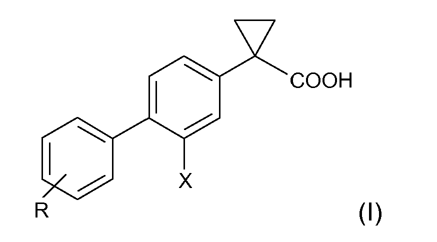

The invention also relates to intermediates useful in said process.

### BACKGROUND OF THE INVENTION

Alzheimer's disease is a neurodegenerative disorder characterized from a histopathologic point of view by a diffuse presence of extracellular and perivascular neuritic plaques and intracellular neurofibrillary tangles in the cerebral parenchyma of Alzheimer patients.

Neuritic plaques are mainly composed of aggregates of a protein with 39-43 amino acid residues known as β-amyloid (βA), and, depending on the numbers of amino acids, Aβ₃₉, Aβ₄₀, Aβ₄₂ and Aβ₄₃.

Compounds have been reported which can reduce the production of the most neurotoxic isoform of β-amyloid, namely the form containing 42 amino acids (Aβ₄₂), through their interaction with a macromolecular/multiprotein enzymatic complex with aspartyl-protease activity, known as γ-secretase.

In particular, WO 2004/074232 discloses derivatives of 1-(2-halobiphenyl-4-yl)-cyclopropanecarboxylic acid capable of modulating γ-secretase activity without affecting other important metabolic processes such as cyclooxygenase-enzymes activity, having general formula (I) 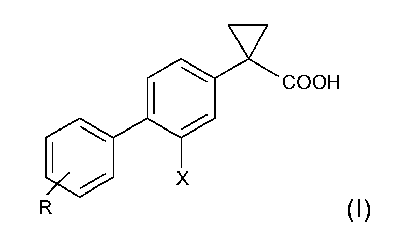 wherein X and R are defined below.

The key intermediate step of the preparation of said compounds is the Suzuki reaction between a suitable phenylboronic acid or an ester thereof with a 3,4-dihalo-cyclopropanecarboxylic acid.

In WO 2004/074232, 3,4-dihalo-cyclopropanecarboxylic acid is obtained starting from 3,4-dihalo-toluene which is transformed into the corresponding benzylbromide by radical bromination in carbon tetrachloride (CCl₄); the resulting bromide is transformed into the 3,4-dihalophenylacetonitrile; the latter is reacted with 1,2 dibromoethane to give the corresponding 3,4-dihalophenylcyclopropanenitrile which is finally hydrolyzed to the desired 3,4-dihalo-cyclopropanecarboxylic.

However, the process disclosed in WO 2004/074232 gives a low overall yield (12-14%) and suffers from severe restrictions for the industrial use.

For example, the radical bromination step yields a significant amount of the bis-halogenated side-product, and involves the use of CCl₄ which is highly toxic and also both ozone-depleting and a greenhouse gas.

In addition, the final Suzuki coupling reaction has a poor yield and the resulting product is difficult to purify by crystallization without a loss of yield. For example, silica gel chromatography has been used for such purification, but its scale-up is tedious and requires large volumes of solvents.

Therefore the present invention concerns a process for the preparation of derivatives of 1-(2-halobiphenyl-4-yl)-cyclopropanecarboxylic acid of formula (I) alternative to that disclosed in WO 2004/074232 and which does not have all the aforementioned drawbacks.

The object of the invention is achieved by carrying out the Suzuki reaction as the first step.

Moreover, different conditions for improving the yield of the other steps have been introduced, in particular the radical bromination step.

The process on the invention turned out to be more efficient, especially for large scale production, providing higher yields in the compounds of formula (I) in high chemical purity without the need for a chromatographic purification step.

### SUMMARY OF THE INVENTION

The present invention concerns a process for preparing a compound of general formula (I) 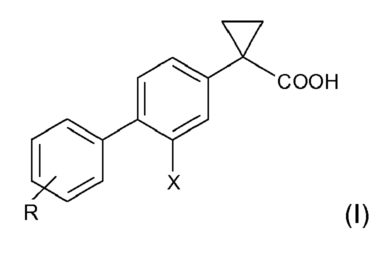 wherein
X is a halogen atom, preferably fluorine;
R represents one or more groups independently selected from:
- halogen atoms, preferably chlorine;
- CF₃;
- CH=CH₂;
- CN;
- CH₂OH;
- NO₂;
- methylenedioxy;
- ethylenedioxy;
- cycloalkyl, preferably C₃-C₆ cycloalkyl;
- phenyl;
- OR₁ or NHCOR₁ wherein R₁ is selected from the group consisting of CF₃, alkenyl, alkynyl, benzyl, and phenyl;
- SR₂, SOR₂ or COR₂ wherein R₂ is alkyl;
and acceptable salts thereof,
said process comprising the following steps:
(i) reacting a compound of formula (II) wherein X is defined as above and X' is chlorine, bromine, iodine or a triflate group (CF₃SO₃) with a compound of formula (III) wherein R is defined as above to form a compound of formula (IV);
(ii) submitting a compound of formula (IV) to radical bromination to form a compound of formula (V);
iii) transforming a compound of formula (V) into the corresponding nitrile derivative of formula (VI);
iv) reacting a compound of formula (VI) with 1,2-dibromoethane to form a compound of formula (VII); and
v) hydrolyzing a compound of formula (VII) to obtain a compound of formula (I).

Advantageously, the radical bromination is carried out with N-bromosuccinimide (NBS) in the presence of a catalytic amount of benzoyl peroxide [PhCOO)₂] and acetonitrile as a solvent.

The invention is also directed to the compound (VII), which has been obtained as stable intermediate.

The invention further concerns a process for preparing a pharmaceutical composition, said process comprising steps (i) -(v) and an additional step (vi) comprising admixture of one or more pharmaceutically acceptable excipients.

### DEFINITIONS

Terms used in the specification have the following meanings:
The term "halogen atoms" includes fluorine, chlorine, bromine, and iodine.
"Alkyl" means straight chain or branched C₁-C₄ alkyl, such as methyl, ethyl, n-propyl, isopropyl, n-butyl, isobutyl, sec-butyl, tert-butyl.
"Alkenyl" means straight chain or branched C₂-C₆ alkenyl, such as vinyl, 1-propenyl, 2-propenyl, 1-butenyl, isobutenyl, or straight-or branched-pentenyl and hexenyl.
"Alkynyl" is to be construed in an analogous manner.
"Cycloalkyl" means a cyclic non-aromatic hydrocarbon group containing from 3 to 8 carbon atoms. Examples include cyclopropyl, cyclobutyl, cyclopentyl, cyclohexyl and cycloheptyl.
"Saturated heterocyclic" means a saturated heterocyclic group having at least 4 carbon atoms and at least one heteroatom, preferably from one to four heteroatoms selected from nitrogen, oxygen and sulfur. Examples include piperidyl or tetrahydrofuryl.

### DETAILED DESCRIPTION OF THE INVENTION

The present invention provides a process for preparing a compound of general formula (I) according to scheme 1. 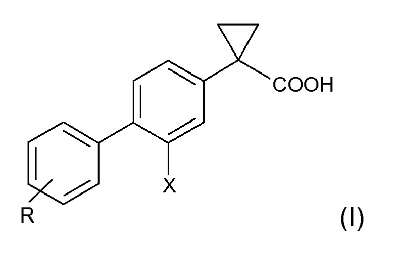 wherein
X and R are as defined above.

When R is cycloalkyl, said ring is optionally substituted with one or more groups independently selected from alkyl, CF₃, OH and oxo groups.

Preferably the cycloalkyl group is C₃-C₆ cycloalkyl.

When R is phenyl, said ring is optionally substituted with one or more groups independently selected from halogen atoms, CF₃, OCF₃, OH, alkyl and a saturated heterocyclic.

The saturated heterocyclic group is preferably a 5- or 6- membered monocyclic ring having one or two nitrogen atoms or one nitrogen atom and one oxygen atom such as pyrrolidine, imidazolidine and isoxazolidine.

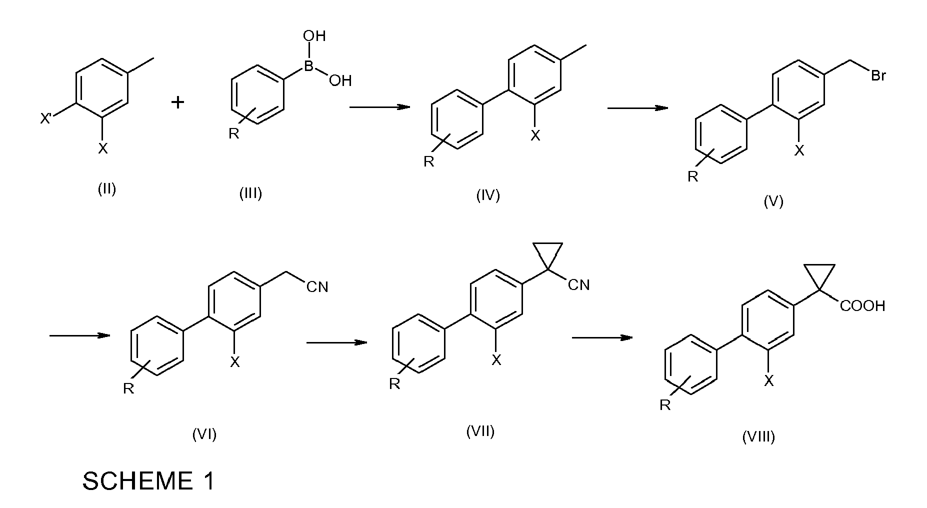

In the first step (step i), a compound having formula (II), wherein X is a halogen atom, preferably fluorine and X' is selected form the group consisting of chlorine, bromine, iodine and a CF₃SO₃ group (triflate), is reacted with a phenyl boronic acid of formula (III) wherein R represents one or more groups independently selected from halogen atoms, preferably chlorine; CF₃; CH=CH₂; CN; CH₂OH; NO₂; methylenedioxy; ethylenedioxy; cycloalkyl; phenyl; OR₁ or NHCOR₁ wherein R₁ is selected from the group consisting of CF₃, alkenyl, alkynyl; benzyl; phenyl; SR₂, SOR₂ and COR₂ wherein R₂ is alkyl.

The compounds of formula (II) and (III) are commercially available or may be prepared according to known methods.

Preferably the reaction, known as Suzuki reaction or Miyaura-Suzuki reaction, is carried out using 4-bromo-3-fluoro-toluene as the compound of formula (II) and 3,4-dichloro-phenylboronic acid as the compound of formula (III).

Said reaction, which relies on a palladium catalyst, may also occur using alkyl boronic esters instead of boronic acids.

Advantageously, any palladium catalyst such as tetrakis(triphenylphosphine)palladium [Pd(PPh)₃], palladium on activated charcoal also known as Palladium on Carbon (Pd on C), palladium on alumina, may be used as catalyst. Preferably Pd on C is used as it is less expensive and easier to handle.

Generally step (i) is carried out in the presence of an organic solvent. Organic solvents which may be advantageously used include ethanol, acetone, tetrahydrofuran (THF), isopropyl alcohol, N-methylpyrrolidone (NMP), dioxane and mixtures thereof with water. A combination of organic solvents may also be used.

Generally, the reaction is carried out at the solvent reflux temperature.

When Pd(PPh)₃ is used, the preferred solvent is a mixture of dioxane/water 2:1 v/v, while, when Pd/C is used, the preferred solvent is ethanol.

Preferably, step (i) is conducted in the presence of a base.

Bases which may be advantageously used include Na₂CO₃, K₂CO₃, K₃PO₄, Cs₂CO₃, NaOH. and KOH. The preferred base is Na₂CO₃.

Optionally additives such as triphenylphosphine (P(Ph₃)), polymethylhydrosiloxane (PMHS), tetrabutylammonium bromide (TBAB), 1,4-diazabicyclo[2.2.2]octane (DABCO, or Nal may be added to the reaction medium.

Preferably, step (i) is carried out in a slight molar excess of the compound (III) with respect to compound (II).

The preferred conditions for the reaction of step (i) are:
- solvent: 20 volumes ethanol;
- base: 2 equivalents Na₂CO₃;
- catalyst: 13% w/w Pd on C 10%.
- temperature: reflux.

Generally, a compound of formula (IV) is obtained in a yield higher than 70%, preferably higher than 80%.

The compound of formula (IV) is preferably 3',4'-dichloro-2-fluoro-4-methyl-biphenyl.

In the second step (step ii), a compound of formula (IV) is submitted to radical bromination to form a compound of formula (V) wherein X and R are as defined above.

Compound (IV) may be as a crude product or may be previously crystallised according to standard procedures.

Advantageously the radical bromination is carried out with N-bromosuccinimide (NBS) in the presence of a catalytic amount of benzoyl peroxide [PhCOO)₂] and acetonitrile as a solvent. Generally, the reaction is carried out at the solvent reflux temperature.

Preferably, in order to minimise the formation of dibrominated product, step (ii) is carried out with a slight excess of NBS, preferably 1.05 mole equivalents to 1 mole equivalents of compound (IV), and in the presence of 0.04 equivalents of PhCOOO₂.

Generally the compound of formula (V), which is preferably 3',4'-dichloro-2-fluoro-4-bromomethyl-biphenyl, is obtained in a yield higher than 85%, preferably higher than 90%.

Optionally, the compound of formula (V) may be further purified by crystallization according to standard procedures.

In the third step (step iii), a compound of formula (V) is transformed into the corresponding nitrile derivative of formula (VI) wherein X and R are as defined above.

Sodium cyanide or another suitable salt may be used.

Advantageously, step (iii) is carried out in an organic solvent such as ethanol or acetonitrile, preferably ethanol.

The temperature used in step (iii) is preferably from about 20°C to about 60° C, more preferably between about 40°C and about 50°C.

Preferably, step (iii) is conducted with a molar excess of sodium cyanide. Advantageously, between 1.2 mole equivalent and 1.0 mole equivalent of sodium cyanide, and preferably 1.05 mole equivalent to 1 equivalent of compound (V), is used.

Generally, the compound of formula (VI), which is preferably 3',4'-dichloro-2-fluoro-4-cyanomethyl-biphenyl, is obtained in a yield higher than 50%, preferably of about 55-60%.

Optionally, said compound may be further purified by crystallization according to standard procedures, preferably by slurrying in ethanol.

In the fourth step (iv), a compound of formula (VI) is reacted with 1,2-dibromoethane to form a compound of formula (VII) wherein X and R are as defined above.

Advantageously, step (iv) is conducted in an organic solvent such as ethanol or acetonitrile or mixtures thereof with water.

Preferably said cyclopropanation is carried out as a phase transfer catalysis reaction in the presence of 30% NaOH and tetrabutylammonium chloride (TBAC) or tetrabutylammonium bromide (TBAB).

The temperature in step (iv) is preferably maintained from about 20°C to about 50°C.

Generally, the compound of formula (VII), which is preferably 1-(3',4'-dichloro-2-fluoro[1,1'-biphenyl]-4-yl)-cyclopropanenitrile, is obtained in a yield higher than 60%, preferably of about 65-70%.

Optionally, said compound may be further purified by crystallization according to standard procedures, preferably using heptane as crystallization solvent.

In the fifth step (step v), a compound of formula (VII) is hydrolized to obtain the desired compound of formula (I) according to known methods.

Preferably, the hydrolysis is carried out in a methanol/water mixture in the presence of a strong base, preferably KOH under reflux.

Generally the compound of formula (I), which is preferably 1-(3',4'-dichloro-2-fluoro[1,1'-biphenyl]-4-yl)-cyclopropanecarboxylic acid, is obtained in a yield higher than 65%.

The compounds of formula (I) may be washed, filtered and isolated by various known techniques. They may be further purified by crystallization according to standard procedures and are obtained with a high chemical purity, e.g. higher than 95% without using final purification by chromatography.

Crystallization from a mixture of heptane and isopropyl alcohol is especially preferred.

The overall yield of the process is usually at least 20%, preferably equal to or higher than 25%, more preferably higher than 30%.

In a preferred embodiment, the invention provides a process for the preparation of a compound of formula (I) wherein X is fluorine and R is chlorine.

In a more preferred embodiment, the invention provides a process for preparing 1-(3',4'-dichloro-2-fluoro[1,1'-biphenyl]-4-yl)-cyclopropanecarboxylic acid having formula (Ia)

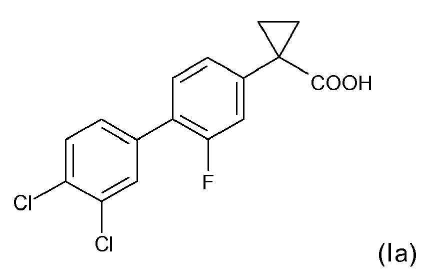

The obtained compound (I) may be further transformed into the corresponding pharmaceutically acceptable salts according to various known techniques.

Pharmaceutically acceptable salts include those in which the acidic function is reacted with an appropriate base to form, e.g., sodium, potassium, calcium, magnesium, and ammonium salts.

The compounds of formula (I) obtained by the process of the invention may be used in the preparation of pharmaceutical compositions for the treatment and/or the prevention of neurodegenerative diseases such as Alzheimer's disease.

Said pharmaceutical compositions, preferably for the oral use, comprise at least one compound of formula (I) in admixture with pharmaceutically acceptable excipients and/or carriers, for example those described in Remington's Pharmaceutical Sciences Handbook, XVII Ed., Mack Pub., N.Y., U.S.A.

The invention is illustrated in greater detail in the following Examples.

### Example 1

### Preparation of 3',4'-dichloro-2-fluoro-4-methyl-biphenyl

3-Fluoro-4-bromotoluene (50 g, 0.265 mol) and 3,4-dichlorophenylboronic acid (53 g, 0.278 mol) are dissolved in ethanol (970 ml) and sodium carbonate (56.1 g, 0.529 mol) is added. Palladium 10% on charcoal (6.6 g) is added, and the mixture is refluxed for 4 hours under nitrogen atmosphere. The reaction mixture is cooled, filtered and concentrated, isopropyl acetate (250 ml) is added, and then the solution is concentrated again. The residue is dissolved in isopropyl acetate (250 ml) and 1M sodium hydroxide (250 ml). The organic phase is separated, washed with water (125 ml), neutralized with 3 M hydrogen chloride, washed with brine (250 ml) and concentrated.

The residue is added with acetonitrile/water 1/1 v/v (150 ml), heated to 40°C to dissolution, then cooled to 0-5°C, and stirred for 30 min at this temperature.

The compound 3',4'-dichloro-2-fluoro-4-methyl-biphenyl crystallizes as a powder.

It is filtered, washed with acetonitrile/water 1/1 v/v (25 ml) and dried at 40°C (56 g, 86% yield).
HPLC-UV purity (210 nm): 95.0%
¹H NMR (DMSO-d6, 300 MHz): 7.73 (m, 2H); 7.49 (m, 2H); 7.14 (m, 2H); 2.36 (s, 3H)

### Example 2

### Preparation of 3',4'-dichloro-2-fluoro-4-bromomethyl-biphenyl

3',4'-Dichloro-2-fluoro-4-methyl-biphenyl (29 g, 0.114 mol), N-bromosuccinimide (21.2 g, 0.119 mol), benzoyl peroxide (1.4 g, 0.004 mol) are dissolved in acetonitrile (190 ml).

The mixture is refluxed for 3 hours, then cooled, added with a solution of sodium sulfite (2.2 g) in water (54 ml), stirred for 30 min and then rested to separate the phases.

The lower aqueous phase is separated and extracted with dichloromethane (29 ml).

The upper phase is concentrated under vacuum, added with water (10 ml) and dichloromethane (58 ml), and stirred. The organic phases are separated and combined, washed twice with water (29 ml), and concentrated under vacuum.

The compound 3',4'-dichloro-2-fluoro-4-bromomethyl-biphenyl is isolated as an orange oil (35.7 g, 94% yield).
HPLC-UV purity (250 nm): 77.1 %
¹H NMR (DMSO-d6, 300 MHz): 7.87-7.12 (m, 6H); 4.76 (s, 2H)

### Example 3

### Preparation of 3',4'-dichloro-2-fluoro-4-cyanomethyl-biphenyl

3',4'-Dichloro-2-fluoro-4-bromomethyl-biphenyl (35.0 g, 0.105 mol) and sodium cyanide (5.4 g, 0.110 mol) are dissolved in a mixture of ethanol (228 ml) and water (25 ml), then heated at 50°C for 3 hours. The solution is concentrated under vacuum and the residue is suspended in ethanol/water 1/1 v/v (35 ml) and cooled at 0-5°C for 30 min.

The obtained solid is filtered and dried at 40°C under vacuum. The crude product is suspended in ethanol (56 ml) at 20-25°C for 30 min, filtered and dried at 40°C under vacuum.

The compound 3',4'-dichloro-2-fluoro-4-cyanomethyl-biphenyl is obtained as a light brown powder (16.8 g, 57% yield).
HPLC-UV purity (250 nm): 92.3%.
¹H NMR (DMSO-d6, 300 MHz): 7.78 (m, 2H); 7.60 (m, 2H); 7.34 (m, 2H); 4.14 (s, 1 H)

### Example 4

### Preparation of 1-(3',4'-dichloro-2-fluoro[1,1'-biphenyl]-4-yl)-cyclopropanenitrile

3',4'-Dichloro-2-fluoro-4-cyanomethyl-biphenyl (9.0 g, 0.032 mol), 1,2-dibromomethane (9.0 g, 0.048 mol), tetrabutylammonium chloride (1.2 g, 0.043 mol), toluene (60 ml) and water (9 ml) are loaded in a reactor.

30% Aq. sodium hydroxide (60 g, 0.45 mol) is dropped over 30 min at 20-25°C and the reaction mixture is stirred for 6 hours. The organic phase is separated, washed with water (12 ml), with 3 M aq. hydrogen chloride (36 ml) and finally with water (12 ml).

The solution is concentrated, then n-heptane (18 ml) is added at 80°C.

The solution is cooled to 0-5°C and stirred for 30 min.

The product crystallized from the solution is filtered, washed with cold n-heptane (5 ml) and dried at 40°C under vacuum.

The compound 1-(3',4'-dichloro-2-fluoro[1,1'-biphenyl]-4-yl)-cyclopropanenitrile is obtained as a yellow powder (6.4 g, 65% yield).
HPLC-UV purity (250 nm): 98.2%.
¹H NMR (DMSO-d6, 300 MHz): 7.78 (m, 2H); 7.60 (m, 2H); 7.30 (m, 2H); 1.84 (m, 2H); 1.63(m,2H).

### Example 5

### Preparation of 1-(3',4'-dichloro-2-fluoro[1,1'-biphenyl]-4-yl)-cyclopropane carboxylic acid

1-(3',4'-Dichloro-2-fluoro[1,1'-biphenyl]-4-yl)-cyclopropanenitrile (14.3 g, 0.047 mol) is dissolved in a mixture of methanol (143 ml) and water (71.5 ml), potassium hydroxide (35.1 g, 0.563 mol) is added portionwise, and the mixture is refluxed for 48 hours.

The reaction mixture is cooled and poured in a solution of 36% aqueous hydrogen chloride (57 ml) in water (57 ml) at 20-25°C. The suspension is stirred and filtered; the solid is repeatedly washed with water and dried at 40°C under vacuum. The crude product is dissolved in refluxing 2-propanol (178 ml), the solution is added with activated carbon (0.3 g), stirred at reflux and filtered, concentrated and added with n-heptane (116 ml). The hot solution is cooled to 0-5°C and the crystallized solid is filtered, washed with 2-propanol and dried at 40°C under vacuum.

The compound 1-(3',4'-dichloro-2-fluoro[1,1'-biphenyl]-4-yl)-cyclopropanecarboxylic acid is obtained as a white powder (10.3 g, 68% yield).
HPLC-UV purity (255 nm): 99.8%
¹H NMR (DMSO-d6, 300 MHz): 12.51 (bs, 1H); 7.78 (m, 2H); 7.54 (m, 2H); 7.30 (m, 2H); 1.48 (m, 2H); 1.22 (m, 2H)
MS (ESI-, 40 V): 323 (M-); 279.
Melting range: 199-200°C.

## Claims

1. A process for preparing a compound of general formula (I) 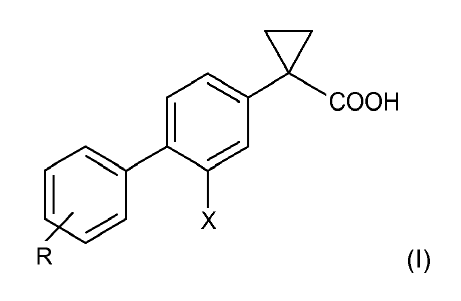 wherein
X is a halogen atom;
R represents one or more groups independently selected from:
- halogen atoms;
- CF₃;
- CH=CH₂;
- CN;
- CH₂OH;
- NO₂;
- methylenedioxy;
- ethylenedioxy;
- cycloalkyl;
- phenyl;
- OR₁ or NHCOR₁ wherein R₁ is selected from the group consisting of CF₃, alkenyl, alkynyl, benzyl, and phenyl;
- SR₂, SOR₂ or COR₂ wherein R₂ is alkyl; and
the pharmaceutically acceptable salts thereof,
said process comprising the following steps:
(i) reacting a compound of formula (II) 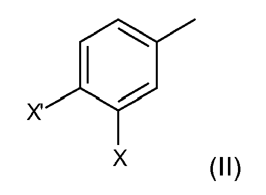 wherein X is defined as above and X' is selected from the group consisting of chlorine, bromine, iodine and a triflate group (CF₃SO₃)
with a compound of formula (III) 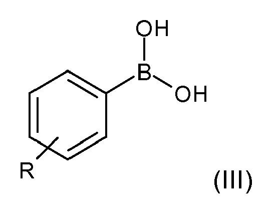 wherein R is defined as above to form a compound of formula (IV) 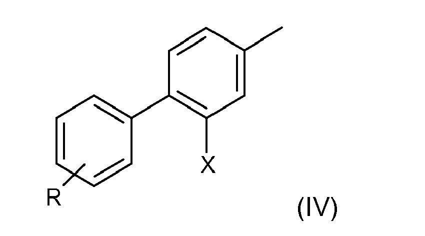
(ii) submitting a compound of formula (IV) to radical brominaton to form a compound of formula (V) 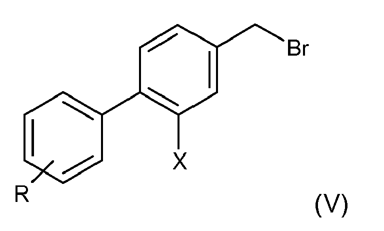
iii) transforming a compound of formula (V) into the corresponding nitrile derivative of formula (VI) 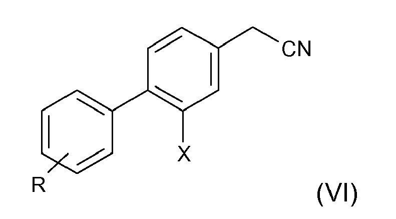
iv) reacting a compound of formula (VI) with 1,2-dibromoethane to form a compound of formula (VII) 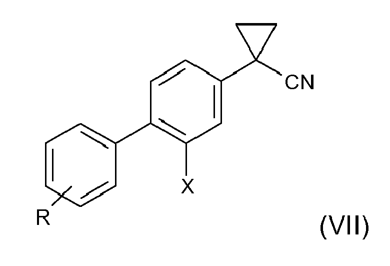 and
v) hydrolyzing a compound of formula (VII) to obtain a compound of formula (I).

2. The process as claimed in claim 1 further comprising the steps of isolating and crystallising the compound of formula (I).

3. The process according to claim 2 wherein crystallization is carried out by using a mixture of heptane and isopropyl alcohol.

4. The process as claimed in claim 1 or 2 wherein step (i) is carried out in the presence of a palladium catalyst selected from the group consisting of tetrakis(triphenylphosphine)palladium, palladium on activated charcoal, and palladium on alumina.

5. The process as claimed in claim 3 or 4 wherein the palladium catalyst is palladium on activated charcoal.

6. The process as claimed in any one of claims 1 to 3 wherein step (ii) is carried with N-bromosuccinimide in the presence of a catalytic amount of benzoyl peroxide using acetonitrile as a solvent.

7. The process as claimed in any one of the preceding claims wherein X is fluorine and R is a halogen atom.

8. The process as claimed in claim 7 wherein the halogen atom is chlorine.

9. The process as claimed in any of the preceding claims comprising the following steps:
(i) reacting 4-bromo-3-fluoro-toluene with 3,4-dichlorophenylboronic acid to form 3',4'-dichloro-2-fluoro-4-bromomethyl-biphenyl;
(ii) submitting 3',4'-dichloro-2-fluoro-4-bromomethyl-biphenyl to radical bromination to form 3',4'-dichloro-2-fluoro-4-bromomethyl-biphenyl;
iii) transforming 3',4'-dichloro-2-fluoro-4-bromomethyl-biphenyl into the corresponding 3',4'-dichloro-2-fluoro-4-cyanomethyl-biphenyl;
iv) reacting 3',4'-dichloro-2-fluoro-4-cyanomethyl-biphenyl with 1,2-dibromoethane to form 1-(3',4'-dichloro-2-fluoro[1,1'-biphenyl]-4-yl)-cyclopropanenitrile; and
v) hydrolyzing 1-(3',4'-dichloro-2-fluoro[1,1'-biphenyl]-4-yl)-cyclopropanenitrile to obtain 1-(3',4'-dichloro-2-fluoro[1,1'-biphenyl]-4-yl)-cyclopropanecarboxylic acid.

10. A compound of formula (VII) wherein
X is a halogen atom; and
R represents one or more groups independently selected from the group consisting of halogen atoms; CF₃; CH=CH₂; CN; CH₂OH; NO₂; methylenedioxy; ethylenedioxy; cycloalkyl; phenyl; OR₁ or NHCOR₁ wherein R₁ is selected from the group consisting of CF₃, alkenyl, alkynyl; benzyl; and phenyl; and SR₂, SOR₂ or COR₂ wherein R₂ is alkyl.

11. The compound as claimed in claim 10 which is 1-(3',4'-dichloro-2-fluoro[1,1'-biphenyl]-4-yl)-cyclopropanenitrile.

12. The process for preparing a pharmaceutical composition comprising steps (i) -(v) of claim 1 and an additional step (vi) comprising admixture of the obtained compound with one or more pharmaceutically acceptable excipients.
